(19) Europäisches Patentamt
European Patent Office
Office européen des brevets

(11) **EP 2 177 500 A1**

(12) **EUROPEAN PATENT APPLICATION**
published in accordance with Art. 153(4) EPC

(43) Date of publication:
**21.04.2010 Bulletin 2010/16**

(51) Int Cl.:
*C07C 51/235* (2006.01)    *C07C 45/35* (2006.01)
*C07C 47/22* (2006.01)    *C07C 57/055* (2006.01)
*C07B 61/00* (2006.01)

(21) Application number: **08778352.8**

(22) Date of filing: **18.07.2008**

(86) International application number:
**PCT/JP2008/063472**

(87) International publication number:
**WO 2009/017074 (05.02.2009 Gazette 2009/06)**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(30) Priority: **27.07.2007 JP 2007195322**

(71) Applicant: **Nippon Shokubai Co., Ltd.
Osaka-shi, Osaka 541-0043 (JP)**

(72) Inventors:
• **TANIMOTO, Michio
Himeji-shi
Hyogo 671-1214 (JP)**
• **HAKOZAKI, Nobuyuki
Himeji-shi
Hyogo 671-1211 (JP)**

(74) Representative: **Albrecht, Thomas
Kraus & Weisert
Patent- und Rechtsanwälte
Thomas-Wimmer-Ring 15
80539 München (DE)**

(54) **PROCESS FOR PRODUCING ACRYLIC ACID BY TWO-STAGE CATALYTIC VAPOR-PHASE OXIDATION**

(57)    As an improvement in the production method of acrylic acid by two-stage catalytic vapor-phase oxidation of propylene comprising catalytic vapor-phase oxidation of a propylene-containing gas at a first reactor to produce an acrolein-containing gas, and successive catalytic vapor-phase oxidation of the obtained product gas to produce acrylic acid, a process which enables acrylic acid production on an industrial scale for a long period, with stability at high yield is offered. This process is **characterized in that** a filler formed of a solid inert material is disposed at a cooling part which is provided on the downstream side to the direction of gas flow through the catalyst layer in the first reactor and/or on the gas outlet side of the first reactor, in such a way that the voidage in the filler becomes 45 - 99%.

EP 2 177 500 A1

**Description**

Technical Field

This invention relates to a process for producing acrylic acid by two-stage catalytic vapor-phase oxidation of propylene.

Background Art

Acrylic acid is an industrially important starting material for various synthetic resins, paints, plasticizing agents and the like. Its importance is still increasing in these years as a starting material for water absorbent resins, and the demand for acrylic acid also tends to increase.

For preparation of acrylic acid, two-stage catalytic vapor-phase oxidation of propylene is the commonest and is broadly and industrially practiced. As an embodiment of this method, a production process of acrylic acid comprising catalytic vapor-phase oxidation of starting propylene at a first fixed bed reactor (which is hereafter referred to as "the first reactor") loaded with a catalyst for converting propylene to acrolein, to produce acrolein, and subsequent catalytic vapor-phase oxidation of the resulting acrolein at a second fixed bed reactor ("the second reactor") which is loaded with a catalyst for converting the acrolein to acrylic acid, is known.

Concerning such a process for producing acrylic acid by two-stage catalytic vapor-phase oxidation of propylene, various proposals have been made to produce the product at a higher yield stably over a prolonged period.

For example, JP Sho 55(1980)-102536A discloses that the use of a gaseous mixture of the gas produced at the first reactor and a waste gas which remains after separating acrylic acid from the gas produced at the second reactor by condensation and to which air or gaseous oxygen was added, as the gas supply to the second reactor enables efficient operation and increases safety of the operation. JP Sho 62(1987)-17579B discloses inhibition of autoxidation of acrolein by providing a cooling zone composed of a layer of a solid inert substance, adjacent to the downstream side of the catalyst layer loaded in the first reactor. JP Hei 1(1989)-165543A discloses insertion of rod-like or platy insert into the space at the gas inlet part of the second reactor in such a way to make the voidage in the tube 40- 99%, to prevent plugging of the catalyst layer loaded in the second reactor by the by-product. Also JP Hei 6(1994)-262081A and Hei 6-263689A disclose that the solid organic matters caused by impurities in the gas or by-products of the reaction can be safely and efficiently removed by contacting them with a gas which contains molecular oxygen and steam at prescribed temperatures. Furthermore, JP 2007-509884A discloses the treatment of a catalyst for producing acrolein from propylene with a gas mixture comprising molecular oxygen, an inert gas and optionally steam, at a predetermined temperature, at least once in a calendar year enables long-term operation.

Disclosure of the Invention

Acrylic acid is presently produced at a scale of several millions of tons per year, however, and an improvement in its yield even by 0.1% on industrial scale economically bears very substantial significance. It is all the more so when the production can be carried out stably over a long term. While all of the production processes as introduced in the above achieve the intended improvements in the yield of acrylic acid or a long-term continuous operation, there is still more room for improvement left, when the increase in demand for acrylic acid in recent years is considered.

While the processes as described in JP Sho 55-102536A and JP Sho 62-17579B enable production of acrylic acid at high efficiency by inhibiting autoxidation of acrolein, they entirely fail to take into consideration the organic matters or carbides attributable to impurities in the starting material or by-products of the reaction. In Examples of these known publications, no evaluation whatsoever is given as to long-term stable production of acrylic acid.

According to the process of JP Hei 1-165543A, it is made possible to prevent plugging of the second reactor catalyst layer with the by-products to a certain extent, by inserting a metallic or ceramic platy insert into the inlet part of the second reactor. However, the use of such a metallic or ceramic platy insert is bound to invite not a little adhesion or deposition of the organic matters or carbides to and on the catalyst, even though the plugging itself could be prevented. Also because this known process gives no consideration for prevention of autoxidation of acrolein and adhesion or deposition of the organic matters or carbides to or on the piping connecting the outlet part of the first reactor and the inlet part of the second reactor, in high probability deterioration in catalytic performance will be caused by adhesion and deposition of the organic matters or carbides, when the reaction is continued over a longer term.

The processes as described in JP Hei 6-262081A and 263689A enable safe and efficient removal of the organic matters or carbides resulting from impurities in the gas or by-products of the reaction. Once plugging of the catalyst layer takes place, however, that effect can no more be obtained. These known publications, furthermore, give no disclosure on controlling adhesion or deposition of such organic matters or carbides to and in the catalyst layer and accompanying plugging of the reaction tube(s).

The process of JP 2007-509884A makes a long-term operation possible in the method of producing acrolein from propylene, by maintaining the difference between the temperature at hot spot of the catalyst layer and the reaction temperature small. This known publication, however, lacks disclosure on adhesion or deposition of the organic matters or carbides, or on their safe and efficient removal. It furthermore gives no disclosure on the second reactor for producing acrylic acid from acrolein.

Accordingly, therefore, the object of the present invention is to offer a process for producing acrylic acid by two-stage catalytic vapor-phase oxidation of propylene at high yield on an industrial scale, stably over a prolonged period.

With the view to accomplish the above object we have engaged in concentrative investigations and discovered, in two-stage catalytic vapor-phase oxidation method of propylene to produce acrylic acid, disposition of a filler formed of a solid inert material at the cooling part which is disposed on the downstream side to the direction of gas flow through the catalyst layer in the first fixed bed reactor and/or on the gas outlet side from the first fixed bed reactor, in such a way that the voidage in the filler becomes 45 - 99%, results in high thermal efficiency and inihibition of autoxidation of acrolein and also of adhesion or deposition of the organic matters or carbides with increased certainty.

Thus, according to the present invention it is made possible to stably produce acrylic acid at higher yield and over a prolonged term, in the two-stage catalytic vapor-phase oxidation method which comprises vapor-phase catalytic oxidation of a propylene-containing gas in the first reactor, and subsequent vapor-phase catalytic oxidation of resulting product gas in the second reactor to produce acrylic acid.

Best Embodiment for Working the Invention

Hereinafter the acrylic acid production process of the present invention by two-stage catalytic vapor-phase oxidation method is explained in detail, but the scope of the invention is not restricted by the following explanation. Embodiments other than those given for example should be understood to be covered by the scope of the present invention, so long as they do not go against the purpose of the invention.

The solid inert material to be loaded in the cooling part disposed at the downstream side to the direction of gas flow through the catalyst layer in the first reactor and/or the gas outlet side of the first reactor is not subject to particular limitation so long as it is capable of making the voidage at the loading time 45 - 99%, preferably 50 - 98%, more preferably 55 - 97%. When the voidage at the packing time of the solid inert material is less than 45%, adhesion or deposition of the organic matters or carbides to and on the cooling part increases, which in certain cases is liable to induce plugging of the reaction tube(s). Whereas, when the voidage exceeds 99%, thermal efficiency at the cooling part is lowered and autoxidation of acrolein is apt to occur with increased ease, leading to increase in the amount of organic matters and carbides supplied to the second reactor, which may induce plugging in the second reactor.

Shape of the solid inert material again is not particularly limited, so long as it can be loaded or accommodated in the reaction tube to satisfy the above-specified voidage at the loading time. For example, it can be, besides Raschig ring, sphere, column or ring, block, rod, plate or wire net. When the material is in the form of rod, it can be used either alone or more than one can be used as bundled together. When it is in plate form, it can be suitably bent, undulated like waves or spirally deformed, or projections may be formed on its surface. Of these, Raschig ring form is preferred.

The constituent of the material also is not particularly limited, so long as it is a substance not substantially participating in the reaction. For example, α-alumina, Alundum, mullite, Carborundum, stainless steel, silicon carbide, steatite, earthen ware, porelain, iron and various kinds of ceramics and the like can be named.

These solid inert materials are not necessarily uniformly loaded throughout the whole of the solid inert material-loaded layer, but from the viewpoint of preventing autoxidation of the acrolein-containing gas and effective cooling, it is preferred to substantially uniformly pack the material throughout the whole of the solid inert material-packed layer. It is also possible to use two or more materials differing in dimensions, shape or constituent in the form of plural layers, or as mixed. In using more than one solid inert materials as above, when used as plural layers, preferably each of the materials is packed substantially uniformly in the respective layers, and when mixed, the composition throughout the whole mixture layer is made substantially uniform.

Because one of the actions or functions of the solid inert material is to quench the acrolein-containing reaction gas to lower its temperature to the range suitable for the oxidation in the second reactor, the inert material-loaded layer should be given a length sufficient for the material to exhibit that action or function. The length should be suitably selected depending on the reaction conditions such as the composition and concentration of starting gas, reaction temperature and so on, and cannot be specified unconditionally. In general terms it is preferably at least 100 mm, in particular, at least 200 mm. When used as plural layers, the ratio between the constituent layers can be suitably and freely designed.

In the present invention, preferably a treating agent for adsorbing and/or absorbing the organic matters and/or carbides is disposed in the cooling part disposed on the upstream side to the direction of gas flow through the catalyst layer in the second reactor and/or the gas inlet part of the second reactor, for preventing adhesion or deposition of the organic matters or carbides to and on the catalyst layer loaded in the second reactor. Adhesion of the organic matters or carbides onto the catalyst invites an increase in pressure drop or plugging of the reaction tube(s). Furthermore, when an aeration treatment of contacting the organic matters or carbides with an oxygen-containing gas at high temperatures is given to remove them, the thermal load incurred by their combustion induces deterioration in the catalytic performance, including a possibility to cause run-away due to the rapid heat generation.

As the treating agent for adsorbing and/or absorbing the organic matters and/or carbides, any that can substantially adsorb and/or absorb the organic matters and/or carbides can be used. For example, those having an organic matter adsorption using crotonaldehyde as the index substance of at least 0.05 mass% are preferred. Constituent(s) of the treating agent are not particularly limited, and oxides, complex oxides or mixtures containing at least one element selected from aluminium, silicon, titanium and zirconium (which are hereafter collectively referred to as "(complex) oxides") can be named by way of example. Of those, complex oxides containing aluminium and silicon are particularly preferred. The treating agent can furthermore comprise alkali metals such as sodium or potassium; alkaline earth metals such as magnesium or calcium; iron, niobium, zinc and the like, which are originated from impurities contained in the starting material, binder, molding assistant and the like.

The treating agent is subject to no particular limitation as to its shape, and may have any optional shape. Spcifically, spherical, columnar, cylindrical, starlike, ring-formed, tabletted or pelletized treating agents, that is, those formed with ordinary tabletting machine, extruder, pelletizer and the like can be used. Two or more kinds of the treating agents differing in dimensions and/or composition may be loaded as plural layers, or mixed.

The use rate of the treating agent can be suitably adjusted according to the kind, specific gravity and shape of the treating agent selected for individual occasions, and to the kind, specific gravity, shape and amount used of the catalyst, and is not particularly limited.

The catalyst to be loaded in the first reactor in the present invention, i.e., the catalyst for converting propylene to acrolein by catalytic vapor-phase oxidation (which may be hereafter referred to simply as "first-stage catalyst") is subject to no particular limitation, and known commonly used oxide catalysts can be used.

As preferred specific examples of the first-stage catalyst, those represented by the following general formula (I):

$$Mo_aBi_bFe_cX1_dX2_eX3_fX4_gO_x \qquad (I)$$

(wherein Mo is molybdenum, Bi is bismuth, Fe is iron, X1 is at least one element selected from cobalt and nickel, X2 is at least one element selected from alkali metal, alkaline earth metal, boron and thallium, X3 is at least one element selected from tungsten, silicon, aluminium, zirconium and titanium, X4 is at least one element selected from phosphorus, tellurium, antimony, tin, cerium, lead, niobium, manganese, arsenic and zinc, and O is oxygen; a, b, c, d, e, f, g and x denote atomic ratios of Mo, Bi, Fe, A, B, C, D and O, respectively, and when a=1.2, b=0.1-10, c=0.1-20, d=2-20, e=0.001-10, f=0-30 and g=0-4, and x is a numerical value determined according to the state of oxidation of each of the elements)
can be named.

The catalyst to be loaded in the second reactor, i.e., the catalyst for converting acrolein to acrylic acid by catalytic vapor-phase oxidation ("second-stage catalyst") again is not subject to any particular limitation, and known commonly used oxidation catalyst can be used.

As preferred specific examples of the second-stage catalyst, oxide catalysts represented by the following general formula (II).

$$Mo_h, V_iW_jY1_kY2_lY3_mY4_nO_y \qquad (II)$$

(wherein Mo is molybdenum, V is vanadium, W is tungsten, Y1 is at least one element selected from antimony, bismuth, chromium, niobium, phosphorus, lead, zinc and tin, Y2 is at least one element selected from copper and iron, Y3 is at least one element selected from alkali metal, alkaline earth metal and thallium, Y4 is at least one element selected from silicon, aluminium, titanium, zirconium, yttrium, rhodium and cerium, and O is oxygen; h, i, j, k, 1, m, n and y denote atomic ratios of Mo, V, W, Y1, Y2, Y3, Y4 and O, respectively, and when h=12, i=2-14, j=0-12, k=0-5, 1=0.01-6, m=0-5 and n=0-10; and y is a numerical value determined according to the state of oxidation of each of the elements)
can be named.

The catalyst can be prepared by known extrusion, tabletting and the like molding processes for giving a prescribed shape to the active component, or by known carrying process to have an optional inert carrier of a prescribed shape carry the active component. Shapes of these molded catalyst and carried catalyst are not particularly limited, which may be spherical, columnar, ring-formed or amorphous. The term, spherical, of course does not strictly mean true spheres but signifies substantially spherical form. This applies also to the terms "columnar" and "ring-formed" as well.

It is unnecessary for the catalysts which are loaded in the first and second reactors to be each of a single kind. For example, the first reactor may be loaded with plural kinds of catalysts differing in activity by the order of their activities, or a part of the catalyst may be diluted with an inert carrier. This also applies to the second reactor.

The reaction temperatures at the first and second reactors are suitably chosen according to such factors including reaction conditions. In general terms, it is 300 - 380°C in the first reactor, and 250 - 350°C in the second reactor. The difference between the reaction temperature at the first reactor and that at the second reactor is normally 10 - 110°C, preferably 30 - 80°C.

The reaction temperature at the first reactor and that at the second reactor substantially correspond to the temperatures of respective heating media at the inlet parts of the respective reactors, and therefore the heating media-inlet temperatures are determined according to the respective reaction temperatures in the first and second reactors as set within the above respective temperature ranges.

The organic matters and/or carbides which are precipitated or adsorbed and/or absorbed onto the solid inert material loaded in the cooling part in the first reactor or onto the treating agent loaded in the cooling part of the second reactor can be safely and efficiently removed by an aeration treatment using a mixed gas containing at least 3 vol% of molecular oxygen and at least 0.5 vol% of steam at 260- 440°C, at a frequency of at least once a year.

In the mixed gas to be used for the aeration treatment, the ratio of molecular oxygen is at least 3 vol%, preferably 4 - 20 vol%, and that of steam is at least 0.5 vol%, preferably 1 - 75 vol%. When the ratio(s) of the molecular oxygen or steam are less than the above lower limit(s), the organic matters or carbides cannot be effectively removed. The necessity caused thereby to suspend the reaction for the aeration treatment over a prolonged period invites economical disadvantages.

While it is sufficient for the mixed gas to contain molecular oxygen and steam within the above ranges, the gas may also contain an inert gas including nitrogen, carbon dioxide and the like, as other component. The ratio of the inert gas in the mixed gas is not higher than 96.5 vol%, preferably not higher than 90 vol%.

In the aeration treatment, the organic matters and/or carbides are contacted with the mixed gas at 260 - 440°C. When the contact treatment temperature is lower than 260°C, sufficient removal of the organic matters and/or carbides cannot be preformed, and when it is higher than 440°C, abnormal heat generation may be induced, which is liable to damage the apparatus. Preferred contact temperature lies within a range of 280 - 420°C. Other conditions for the contact treatment of the organic matters and/or carbides with the mixed gas are subject to no particular limitation. For example, the introduction rate (flow rate) of the mixed gas is suitably determined by capacity limit proper to the apparatus, and the contact time also is suitably determined, the treatment being normally terminated upon disappearance of carbon oxide generation. It is recommendable to carry out this treatment at a frequency of at least once a year. When the aeration treatment is not given over an excessively long period, the organic matters and/or carbides begin to precipitate also on the catalyst layers, leading to a possibility of inducing even clogging of the reaction tubes. Preferred frequency is at least twice a year, more preferably at least three times a year.

In the aeration treatment, the mixed gas may be introduced into the first reactor as connected to the second reactor so that the mixed gas leaving the first reactor is introduced into the second reactor as it is, or the first and second reactors are disconnected to allow introduction of the mixed gas into each of the reactors independently of each other. This aeration treatment is effective also for removing the organic matters and/or carbides adhering to the piping and the like which connect the first reactor and second reactor.

The acrylic acid-containing gas produced of the catalytic vapor-phase oxidation of the present invention is collected by the means known per se, for example, absorption into a solvent such as water or high-boiling hydrophobic organic matter, or direct condensation, as an acrylic acid-containing liquid, which then is purified by known extraction, distillation, crystallization or the like means, to give purified acrylic acid.

When a monomer or monomer mixture containing as its chief component the so obtained purified acrylic acid and/or salt thereof (preferably at least 70 mol%, inter alia, at least 90 mol%) is subjected to crosslinking polymerization using about 0.001 - 5 mol% of a crosslinking agent and about 0.001 - 2 mol% of a radical polymerization initiator, both based on the acrylic acid present, and subsequently to such known procedures as drying, pulverizing and so on, a water-absorbent resin can be obtained.

Hereinafter the present invention is more specifically explained referring to Examples, it being understood that the present invention is under no restriction by the following Examples but can be worked with suitable modifications within the scope conforming to the purpose of the invention. In the following, "mass part" may be simply indicated as "part" for the sake of convenience. Propylene conversion and acrylic acid yield are calculated according to the following equations.

$$\text{Propylene conversion (mol\%)}$$
$$= (\text{mol number of reacted propylene/mol number of supplied}$$
$$\text{propylene}) \times 100$$

$$\text{Acrylic acid yield (mol\%)}$$
$$= (\text{mol number of produced acrylic acid/mol number of supplied}$$
$$\text{propylene}) \times 100$$

Example 1.

[Preparation of first-stage catalyst 1]

In 2000 parts of distilled water, 500 parts of ammonium molybdate was dissolved under heating and stirring (solution A). Separately, 357 parts of cobalt nitrate and 192 parts of nickel nitrate were dissolved in 500 parts of distilled water (solution B). Also separately, 172 parts of ferric nitrate and 195 parts of bismuth nitrate were dissolved in an acidic solution formed by adding 30 parts of conc. nitric acid (65 wt%) to 350 parts of distilled water (solution C). These nitrate solutions (solutions B and C) were added to solution A dropwise, and successively a solution of 2.4 parts of potassium nitrate in 50 parts of distilled water was added. The resulting suspension was evaporated to dryness to form a solid cake, which was calcined at 440°C for about 5 hours. The calcined solid was pulverized to not greater than 250 $\mu$m in size to provide a catalyst powder. Into a centrifugal flow coating apparatus, $\alpha$-alumina spherical carrier of 4 mm in average diameter was added, and into which the catalyst powder together with a 15 wt% aqueous ammonium nitrate solution as the binder was added as being passed through 90°C hot air, to have it carried on the carrier, followed by 6 hours' heat treatment at 470°C in an atmosphere of air. Thus a first-stage catalyst 1 was obtained, in which the composition of the metal elements other than oxygen in the active ingredient (other than the carrier) in terms of atomic ratio was as follows:

$$Mo_{12}Bl_{1.7}Fe_{1.8}Co_{5.2}Ni_{2.8}K_{0.1}.$$

[Preparation of second-stage catalyst 1]

In 2000 parts of distilled water, 350 parts of ammonium paramolybdate, 58 parts of ammonium metavanadate and 53.5 parts of ammonium paratungstate were dissolved under heating and stirring. Separately, in 200 parts of distilled water, 47.9 parts of copper nitrate was dissolved under heating and stirring. The resulting two aqueous solutions were mixed, and to which 12 parts of antimony trioxide was further added to give a suspension. This suspension was evaporated to dryness to make a solid cake. The solid was calcined for about 5 hours at 390°C. The calcined solid was pulverized to not greater than 250 $\mu$m in size to provide a catalyst powder. Into a centrifugal flow coating apparatus, $\alpha$-alumina spherical carrier of 4 mm in average diameter was added, and into which the catalyst powder together with a 15 wt% aqueous ammonium nitrate solution as the binder was added as being passed through 90°C hot air, to have it carried on the carrier, followed by 6 hours' heat treatment at 400°C in an atmosphere of air. Thus a second-stage catalyst 1 was obtained, in which the composition of the metal elements other than oxygen in the active ingredient (other than the carrier) in terms of atomic ratio was as follows:

$$Mo_{12}V_3W_{1.2}Cu_{1.2}Sb_{0.5}.$$

[The first reactor]

A steel reaction tube of 25 mm in inner diameter and 3000 mm in length was set perpendicularly, and from the top of the reaction tube the first-stage catalyst 1 and SUS Raschig rings each 7 mm in outer diameter, 7 mm in length and 0.5 mm in thickness were dropped to load the tube. The layer length of the first-stage catalyst 1 from the bottom of the reaction tube was 2800 mm, that of the Raschig rings thereon was 200 mm, and the voidage at the SUS Raschig ring layer was 95.5%. A jacket for heating medium circulation was disposed outside the reaction tube over its length of 2800 mm from the bottom, and the temperature of the heating medium (reaction temperature) was maintained at 330°C. To let the part of the reaction tube down to 200 mm from the top function as the cooling part, the part was maintained at 260°C with an electric heater.

[The second reactor]

A steel reaction tube of 25 mm in inner diameter and 3000 mm in length was set perpendicularly, and from the top of the reaction tube the second-stage catalyst 1 was dropped to load the tube, with a layer length of 2800 mm. The 200 mm-long part of the reaction tube from the top was not loaded and left as an empty cylindrical part. A jacket for heating medium circulation was disposed over the whole length (3000 mm) of the reaction tube, and the temperature of the heating medium (reaction temperature) was maintained at 260°C.

The outlet from the first reactor (the upper end) and the inlet to the second reactor (the upper end) were connected with a steel pipe of 20 mm in inner diameter and 2000 mm in length, which could be heated externally with an electric heater. The pipe was maintained at 180°C.

[Oxidation reaction]

From the lower part of the first reactor a mixed gas composed of 5.5 vol% of propylene, 10 vol% of oxygen, 25 vol% of steam and 59.5 vol% of nitrogen was introduced as the starting gas at a space velocity to the first-stage catalyst of 1700 h$^{-1}$ (STP), and the reaction gas formed in the first reactor was introduced into the second reactor from its upper part to carry out the vapor-phase catalytic oxidation.

[Aeration treatment]

After 8000 hours of the operation, the reaction was suspended and inside of the reactors was examined. A minor deposition of carbides was observed on the surfaces of the SUS Raschig rings at the cooling part of the first reactor, empty cylindrical part of the reaction tube and the second-stage catalyst at the inlet side of the second reactor. Whereupon the heating medium temperature and the heater temperature at the cooling part of the first reactor were raised to 350°C, and the heating medium temperature for the second reactor was raised to 340°C. From the lower part of the first reactor a mixed gas composed of 15 vol% of oxygen, 50 vol% of steam and 35 vol% of nitrogen was passed at a space velocity of 15 liters per minute (STP) for 30 hours. Thereafter the inside of the reactors was examined and complete removal of the deposited carbides was confirmed. After this treatment, the reaction was continued again.

The result of the reaction was as shown in Table 1.

Example 2

[Preparation of first-stage catalyst 2]

In 2000 parts of distilled water, 500 parts of ammonium molybdate was dissolved under heating and stirring (solution A). Separately, 227 parts of cobalt nitrate and 227 parts of nickel nitrate were dissolved in 500 parts of distilled water (solution B). Also separately, 57.2 parts of ferric nitrate and 114.5 parts of bismuth nitrate were dissolved in an acidic solution formed by adding 30 parts of conc. nitric acid (65 wt%) to 350 parts of distilled water (solution C). These nitrate solutions (solutions B and C) were added to solution A dropwise. Successively, 4.5 parts of borax, 1702 parts of 20 wt% silica sol and 2.4 parts of potassium nitrate were added. The resulting suspension was heated under stirring, and the resulting dry matter was re-dried at 200°C and pulverized. Thus formed powder was tablet-molded into pellets each 5 mm in outer diameter and 4 mm in length. The molded product was calcined at 470°C for 6 hours in an atmosphere of air to give first-stage catalyst 2. The composition of metal elements other than oxygen in the active ingredient of this catalyst in terms of atomic ratio was as follows:

$$Mo_{12}Bi_1Co_{3.3}Ni_{3.3}Fe_{0.6}Na_{0.1}B_{0.2}K_{0.1}Si_{24}.$$

[Preparation of second-stage catalyst 2]

In 2000 parts of distilled water, 350 parts of ammoniuim paramolybdate, 64.1 parts of ammonium metavanadate and 78.5 parts of ammonium paratungstate were dissolved under heating and stirring. Separately, 70.2 parts of copper nitrate was dissolved in 200 parts of distilled water under heating and stirring. The resulting two aqueous solutions were mixed, and to which 44.5 parts of antimony trioxide was added to form a suspension. The suspension was placed in a porcelain evaporator on a hot water bath, and into which spherical silica-alumina carrier of 5 mm in average particle diameter was added, followed by evaporation to dryness under stirring to have the catalytic ingredient adhere onto the carrier. Thus obtained supported product was calcined at 400°C for 6 hours in an atmosphere of air to give second-stage catalyst 2. The composition of metal elements other than oxygen in the active ingredient of this catalyst (excluding the carrier) in terms of atomic ratio was as follows:

$$Mo_{4.16}V_{1.15}W_{0.61}Cu_{0.61}Sb_{0.64}.$$

[Reactors and oxidation reactions]

Loading of the reactors and the oxidation reactions were carried out similarly to Example 1, except that the first-stage catalyst 1 was replaced by the first-stage catalyst 2, the second-stage catalyst 1 was replaced with the second-stage catalyst 2, and steatite of 7 mm in outer diameter, 3 mm in inner diameter and 6 mm in length was loaded in place of the SUS Raschig rings. The voidage in the steatite layer was 66.2%.

After 8000 hours of the operation, the reaction was suspended and inside of the reactors was examined. A minor deposition of carbides was observed on the surfaces of the steatite at the cooling part of the first reactor, empty cylindrical part of the reaction tube and the second-stage catalyst at the inlet side of the second reactor. Upon carrying out the aeration treatment under the same conditions to those in Example 1, the deposited carbides were completely removed. After the treatment, the reaction was continued again.

The result of the reaction was as shown in Table 1.

Example 3

The vapor-phase catalytic oxidation was carried out similarly to Example 1, except that the first-stage catalyst 2 was loaded instead of the first-stage catalyst 1 and ceramic balls of each 7.5 mm in average diameter were loaded instead of the SUS Raschig rings. The voidage in the ceramic ball layer was 45.7%.

After 8000 hours of the operation, the reaction was suspended and inside of the reactors was examined. A minor deposition of carbides was observed on the surfaces of the ceramic balls at the cooling part of the first reactor, empty cylindrical part of the reaction tube and the second-stage catalyst at the inlet side of the second reactor. Upon carrying out the aeration treatment under the same conditions to those in Example 1, the deposited carbides were completely removed. After the treatment, the reaction was continued again.

The result of the reaction was as shown in Table 1.

Comparative Example 1

The vapor-phase catalytic oxidation was carried out similarly to Example 1, except that SUS Raschig rings were not loaded in the first reactor.

After 4000 hours of the operation, the pressure drop increased rapidly, and the reaction was suspended and inside of the reactors was examined. A large amount of carbides was deposited on the empty cylindrical part of the reaction tube and the second-stage catalyst at the inlet part of the second reactor and was about to plug them. When the aeration treatment as in Example 1 was given, rapid heat generation took place at the catalyst layer in the inlet part of the second reactor, and the treatment was discontinued. The carbides, therefore, could be scarcely removed. The reaction was further continued in that state, but the performance was drastically lowered due to degradation of the second-stage catalyst resulted from the heat generation at the time of the aeration treatment.

The result of the reaction was as shown in Table 1.

Comparative Example 2

The vapor-phase catalyst reaction was carried out similarly to Example 3, except that the average diameter of the ceramic balls loaded in the first reactor was changed to 5 mm. The voidage in the ceramic ball layer was 32.7%.

After 6000 hours of the operation, the pressure drop increased rapidly, and the reaction was suspended to check inside of the reactors. A large amount of carbides was deposited on the first-stage catalyst at the outlet part of the first reactor and the ceramic ball layer and was about to plug them. When the aeration treatment as in Example 1 was carried out, rapid heat generation took place at the catalyst layer in the outlet part of the first reactor, and the treatment was discontinued. The carbides, therefore, could be scarcely removed. The reaction was further continued in that state, but the performance was drastically lowered due to degradation of the first-stage catalyst resulted from the heat generation during the aeration, and degradation of the second-stage catalyst caused by introduction of the high-temperature gas into the second reactor.

The result of the reaction was as shown in Table 1.

Example 4

[Preparation of vapor-phase oxidation catalyst]

First-stage catalyst 3 for vapor-phase catalytic oxidation of propylene-containing gas to produce acrolein-containing gas was prepared according to the process as described in Catalyst Preparation Example 1 of JP 2003-251183A. Similarly, second-stage catalyst 3 for vapor-phase catalytic oxidation of acrolein-containing gas to produce acrylic acid was prepared according to the process as described in JP Hei 8(1996)-206505A. The compositions of the metal elements other than oxygen in the active ingredients (other than the carriers) of these catalysts in terms of atomic ratio were as follows:

first-stage catalyst 3
$Mo_{12}Bi_{1.3}Fe_{0.8}Co_5Ni_3Si_2K_{0.08}$
second-stage catalyst 3
$Mo_{12}V_6W_1Cu_{2.2}Sb_{0.5}$.

[Preparation of treating agent]

Sixty(60) mass parts of alumina powder of 20 $\mu$m in average particle diameter and 5 mass parts of methyl cellulose as the binder were put into a kneader and thoroughly mixed. Then 40 mass parts as $SiO_2$ of colloidal silica of 50 nm in average particle diameter was added. Further an adequate amount of water was added and mixed. The resulting mixture was extrusion molded, dried and calcined at 800°C for 2 hours to give ring-formed alumina-silica treating agent of each, on the average, 7 mm in outer diameter, 7.5 mm in length and 2 mm in thickness. The organic matter adsorption of this treating agent using crotonaldehyde as the index substance was 0.3 mass%.

- Organic matter adsorption measurement -

Fifty(50)g of the treating agent was weighed, loaded in a fixed bed flow apparatus and maintained at 350°C. Nitrogen gas was bubbled through crotonaldehyde which was kept at 10°C, and thereafter passed through the loaded apparatus from the upstream side of the treating agent at a rate of 170 ml/min for an hour. After this adsorption treatment the whole amount of the treating agent was heat-treated in air at temperatures up to 500°C. The change in the mass of the treating agent before and after the heat treatment was measured.
The organic matter adsorption was determined according to the following equation:

$$\text{organic matter adsorption (mass\%)}$$
$$= (\text{reduced amount (g)/amount of the treating agent (g)}) \times 100.$$

[Reactors and oxidation]

Example 1 was repeated except that the catalysts used were changed to the first-stage catalyst 3 and second-stage catalyst 3, and that the above treating agent was loaded in the 200 mm-long empty cylindrical part at the gas inlet side of the second reactor.
After 8000 hours of the operation, the reaction was suspended and inside of the reactors was examined. A minor deposition of carbides was observed on the surfaces of the SUS Raschig rings at the cooling part of the first reactor and on the treating agent at the inlet part of the second reactor. Upon carrying out the aeration treatment under the same conditions to those in Example 1, the deposited carbides were completely removed. After the treatment, the reaction was continued again.
The result of the reaction was as shown in Table 1.

Example 5

Example 4 was repeated except that the 200 mm-empty cylindrical part at the gas inlet side of the second reactor was loaded with a metallic sheet, i.e., a 0.4 mm-thick, 17 mm-wide and 280 mm-long SUS sheet as bent in zig-zag manner at an angle of 90° and a pitch of 35 mm, instead of the treating agent.
After 8000 hours of the operation, the reaction was suspended and inside of the reactors was examined. A minor deposition of carbides was observed on the surfaces of the SUS Raschig rings at the cooling part of the first reactor, the SUS metal sheet and the second-stage catalyst at the inlet part of the second reactor. Upon carrying out the aeration treatment under the same conditions to those in Example 1, the deposited carbides were completely removed. After the treatment, the reaction was continued again.

The result of the reaction was as shown in Table 1.

Comparative Example 3

The vapor-phase catalytic oxidation was carried out similarly to Example 4, except that the SUS Raschig rings were not loaded in the first reactor.

After 4000 hours of the operation, the pressure drop increased rapidly, and the reaction was suspended and inside of the reactors was examined. A large amount of carbides was deposited on the treating agent and the second-stage catalyst at the inlet part of the second reactor and was about to plug them. When the aeration treatment as in Example 1 was given, rapid heat generation took place at the inlet part of the catalyst layer in the second reactor, and the treatment was discontinued. The carbides, therefore, could be scarcely removed. The reaction was further continued in that state, but the performance was drastically lowered due to degradation of the second-stage catalyst resulted from the heat generation at the time of the aeration treatment.

The result of the reaction was as shown in Table 1.

Example 6

Example 4 was repeated except that the composition of the gas which was used in the aeration treatment given after 8000 hours of the operation was changed to 2.5 vol% of oxygen and 97.5 vol% of inert gases including nitrogen. This mixed gas was passed at a space velocity of 15 liters/min (STP) for 30 hours, but the deposited carbides could not be sufficiently removed. The treatment time, therefore, was extended by further 30 hours, but still the carbides could not be completely removed. The reaction was resumed, leaving this state as it was.

The result of the reaction was as shown in Table 1.

TABLE 1

| | First reactor cooling part | Voidage (%) | Second reactor inlet part | Operation time (Hr) | Carbides | Propylene conversion (%) | Acrylic acid yield (%) |
|---|---|---|---|---|---|---|---|
| Example 1 | SUS Raschig ring | 95.5 | none | 24 | - | 97.8 | 89.4 |
| | | | | 8000 | very minor amount | 97.0 | 88.6 |
| | | | | After aeration | removed | 97.4 | 88.9 |
| Example 2 | Steatite ring | 66.2 | none | 24 | - | 97.4 | 89.2 |
| | | | | 8000 | very minor amount | 96.5 | 88.2 |
| | | | | After aeration | removed | 96.9 | 88.7 |
| Example 3 | Ceramic ball | 45.7 | none | 24 | - | 97.3 | 89.1 |
| | | | | 8000 | very minor amount | 96.3 | 87.5 |
| | | | | After aeration | removed | 96.8 | 88.6 |
| Comparative Example 1 | None | 100 | none | 24 | - | 97.0 | 88.6 |
| | | | | 8000 | large amount | 95.4 | 85.3 |
| | | | | After aeration | not removed | 93.3 | 82.0 |

(continued)

| | First reactor cooling part | Voidage (%) | Second reactor inlet part | Operation time (Hr) | Carbides | Propylene conversion (%) | Acrylic acid yield (%) |
|---|---|---|---|---|---|---|---|
| Comparative Example 2 | Ceramic ball | 32.7 | none | 24 | - | 97.3 | 89.1 |
| | | | | 8000 | large amount | 94.8 | 84.9 |
| | | | | After aeration | not removed | 92.9 | 81.5 |
| Example 4 | SUS Raschig ring | 95.5 | Treating agent | 24 | - | 98.2 | 90.3 |
| | | | | 8000 | very minor amount | 97.6 | 89.8 |
| | | | | After aeration | removed | 98.2 | 90.4 |
| Example 5 | SUS Raschig ring | 95.5 | SUS wavy metal sheet | 24 | - | 98.2 | 90.2 |
| | | | | 8000 | very minor amount | 97.1 | 88.7 |
| | | | | After aeration | removed | 97.5 | 89.4 |
| Comparative Example 3 | None | 100 | Treating agent | 24 | - | 97.4 | 89.3 |
| | | | | 8000 | large amount | 95.7 | 85.5 |
| | | | | After aeration | not removed | 93.1 | 81.5 |
| Example 6 | SUS Raschig ring | 95.5 | Treating agent | 24 | - | 98.2 | 90.3 |
| | | | | 8000 | very minor amount | 97.5 | 89.7 |
| | | | | After aeration | not completely removed | 97.6 | 89.6 |

Example 7

[Preparation of vapor-phase oxidation catalysts]

The first-stage catalysts 4 and 5 for producing acrolein-containing gas by vapor-phase catalytic oxidation of propylene-containing gas were prepared following the process as described in Example 1 of JP Hei 4(1992)-217932A. Similarly, the second-stage catalysts 4 and 5 for producing acrylic acid by vapor-phase catalytic oxidation of acrolein-containing gas were prepared following the process as described in Example 2 of JP Hei 9(1997)-241209A. The compositions of the metal elements other than oxygen in the active ingredients (other than the carriers) of these catalysts were as follows:

First-stage catalyst 4:

$Mo_{10}W_2Bi_1Fe_1Co_4K_{0.06}Si_{1.5}$ (average diameter 5 mm)

First-stage catalyst 5:

$Mo_{10}W_2Bi_1Fe_1Co_4K_{0.06}Si_{1.5}$ (average diameter 8 mm)

Second-stage catalyst 4:

$Mo_{12}V_4W_{2.5}Cu_2Sr_{0.2}$ (average diameter 5 mm)

Second-stage catalyst 5:

$Mo_{12}V_4W_2Cu_2Sr_{0.2}$ (average diameter 8 mm)

[The first reactor]

A fixed bed shell-and-tube reactor having 13,000 reaction tubes (each 25 mm in diameter and 3000 mm in length) was loaded with the first-stage catalyst 5, first-stage catalyst 4, and SUS Raschig rings of each 7 mm in outer diameter, 7 mm in length and 0.5 mm in thickness, which were successively dropped from the upper part of the reaction tubes by the order stated, to make their respective layer lengths from the bottoms of the reaction tubes as follows: 800 mm of the first-stage catalyst 5 layer, 2000 mm of the first-stage catalyst 4 layer, and 200 mm of the SUS Raschig ring layer. The voidage in the SUS Raschig ring layer was 95.5%. A jacket for the heating medium circulation was disposed outside of the reactor, over the part up to 2800 mm from the bottoms of the reaction tubes. The temperature of the heating medium (reaction temperature) was maintained at 320°C, and the part down to 200 mm from the tops of the reaction tubes was maintained at 260°C to function as the cooling part, by a separately externally disposed jacket for the heating medium circulation.

[The second reactor]

A fixed bed shell-and-tube reactor having 13,000 reaction tubes (each 25 mm in diameter and 3000 mm in length) was loaded with the second-stage catalyst 4, second-stage catalyst 5, and the treating agent as used in Example 4, which were successively dropped from the upper part of the reaction tubes by the order stated, to make their respective layer lengths from the bottoms of the reaction tubes as follows: 2000 mm of the second-stage catalyst 4 layer, 800 mm of the second-stage catalyst 5 layer, and 200 mm of the treating agent layer. Over the whole length of the reaction tube (3000 mm), an external jacket for heating medium circulation was disposed to maintain the temperature of the heating medium (reaction temperature) at 260°C.
The outlet from the first reactor (the upper end) and the inlet of the second reactor (the upper end) were connected with a steel pipe of 500 mm in inner diameter and 4000 mm in length, which could be externally heated with vapor and maintained at 180°C.

[Oxidation reaction]

From the lower part of the first reactor a mixed gas composed of 7 vol% of propylene, 13 vol% of oxygen, 8 vol% of steam and 72 vol% of nitrogen was introduced as the starting gas at a space velocity to the first-stage catalyst of 1500 h$^{-1}$ (STP), and the reaction gas formed in the first reactor was introduced into the second reactor from the upper part thereof, to carry out the vapor-phase catalytic oxidation.

[Aeration treatment]

At every 4000 hours of the operation the reaction was suspended, followed by an aeration treatment. In the aeration treatment, the heating medium temperature for the catalyst layer and that for the catalyst layer at the cooling part, of the first reactor were raised to 350°C, and the heating medium temperature for the catalyst layer of the second reactor was raised to 340°C. From the lower part of the first reactor a mixed gas composed of 10 vol% of oxygen, 50 vol% of steam and 40 vol% of inert gases including nitrogen was passed at a space velocity of 195 m$^3$/min (STP) for 20 hours. After each aeration treatment the reaction was continued, until 16000 hours passed in total. Thereafter the inside of the reactors was checked to find nearly no deposition of carbides.
The result of the reaction was as shown in Table 2.

TABLE 2

| | Operation time (Hr) | Accumulative operation time (Hr) | Propylene conversion (%) | Acrylic acid yield (%) |
|---|---|---|---|---|
| | 48 | 48 | 98.5 | 90.2 |
| Example 7 | 4000 | 4000 | 98.1 | 89.8 |
| | After aeration | | 98.4 | 90.2 |
| | 8000 | 8000 | 97.8 | 89.5 |
| | After aeration | | 98.4 | 90.1 |
| | 12000 | 12000 | 97.5 | 89.1 |
| | After aeration | | 98.3 | 89.9 |
| | 16000 | 16000 | 97.3 | 88.9 |
| | After aeration | | 98.0 | 89.7 |

**Claims**

1. A process for producing acrylic acid by two-stage catalytic vapor-phase oxidation method comprising catalytic vapor-phase oxidation of a pxopylene-containing gas at a first fixed bed reactor which is loaded with a catalyst for converting propylene to acrolein by catalytic vapor-phase oxidation, to produce an acrolein-containing gas, and catalytic vapor-phase oxidation of the formed reaction gas at a second fixed bed reactor which is loaded with a catalyst for converting acrolein to acrylic acid by catalytic vapor-phase oxidation, to produce acrylic acid, the process being **characterized in that** a filler formed of a solid inert material is disposed at a cooling part which is provided on the downstream side to the direction of gas flow through the catalyst layer in the first fixed bed reactor and/or on the gas outlet side of the first fixed bed reactor, in such a way that the voidage in the filler becomes 45 - 99%.

2. A process according to Claim 1, which is **characterized in that** a treating agent for adsorbing and/or absorbing the organic matters and/or carbides is disposed at the cooling part disposed on the upstream side to the direction of gas flow through the catalyst layer in the second reactor and/or the gas inlet part of the second reactor.

3. A process according to Claim 1 or 2, which is **characterized in that** the catalyst to be loaded in the first fixed bed reactor for converting propylene to acrolein by catalytic vapor-phase oxidation is one represented by the following general formula (I):

$$Mo_aBi_bFe_cX1_dX2_eX3_fX4_gO_x \qquad (I)$$

(wherein Mo is molybdenum, Bi is bismuth, Fe is iron, X1 is at least one element selected from cobalt and nickel, X2 is at least one element selected from alkali metal, alkaline earth metal, boron and thallium, X3 is at least one element selected from tungsten, silicon, aluminium, zirconium and titanium, X4 is at least one element selected from phosphorus, tellurium, antimony, tin, cerium, lead, niobium, manganese, arsenic and zinc, and O is oxygen; a, b, c, d, e, f, g and x denote atomic ratios of Mo, Bi, Fe, A, B, C, D and O, respectively, and when a=12, b=0.1-10, c=0.1-20, d=2-20, e=0.001-10, f=0-30 and g=0-4, and x is a numerical value determined according to the state of oxidation of each of the elements).

4. A process according to any of Claims 1 - 3, which is **characterized in that** the catalyst to be loaded in the second fixed bed reactor for converting acrolein to acrylic acid by catalytic vapor-phase oxidation is one represented by the following general formula (II).

$$Mo_h,V_i,W_jY1_kY2_lY3_mY4_nO_y \qquad (II)$$

(wherein Mo is molybdenum, V is vanadium, W is tungsten, Y1 is at least one element selected from antimony, bismuth, chromium, niobium, phosphorus, lead, zinc and tin, Y2 is at least one element selected from copper and iron, Y3 is at least one element selected from alkali metal, alkaline earth metal and thallium, Y4 is at least one element selected from silicon, aluminium, titanium, zirconium, yttrium, rhodium and cerium, and O is oxygen; h, i.

j, k, 1, m, n and y denote atomic ratios of Mo, V, W, Y1, Y2, Y3, Y4 and O, respectively, and when h=12, i=2-14, j=0-12, k=0-5, 1=0.01-6, m=0-5 and n=0-10; and y is a numerical value determined according to the state of oxidation of each of the elements).

5. A process for producing acrylic acid according to any of Claims 1 - 4, which is **characterized in that** the reaction is temporarily suspended and an aeration treatment is performed using a mixed gas containing at least 3 vol% of molecular oxygen and at least 0.5 vol% of steam, at 260 - 440°C, at a frequency of at least once a year.

## INTERNATIONAL SEARCH REPORT

| International application No. |
| --- |
| PCT/JP2008/063472 |

A. CLASSIFICATION OF SUBJECT MATTER
*C07C51/235*(2006.01)i, *C07C45/35*(2006.01)i, *C07C47/22*(2006.01)i,
*C07C57/055*(2006.01)i, *C07B61/00*(2006.01)n

According to International Patent Classification (IPC) or to both national classification and IPC

B. FIELDS SEARCHED

Minimum documentation searched (classification system followed by classification symbols)
C07C51/235, C07C45/35, C07C47/22, C07C57/055, C07B61/00

Documentation searched other than minimum documentation to the extent that such documents are included in the fields searched
Jitsuyo Shinan Koho          1922-1996   Jitsuyo Shinan Toroku Koho   1996-2008
Kokai Jitsuyo Shinan Koho    1971-2008   Toroku Jitsuyo Shinan Koho   1994-2008

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X<br>Y | JP 2003-1094 A  (Nippon Shokubai Co., Ltd.),<br>07 January, 2003 (07.01.03),<br>Par. Nos. [0085], [0087]; examples<br>& US 2007/0003460 A1    & EP 1270065 A1 | 1,3,4<br>2,5 |
| X<br>Y | JP 2001-137689 A  (Nippon Shokubai Co., Ltd.),<br>22 May, 2001 (22.05.01),<br>Par. Nos. [0078], [0079]; examples<br>& US 6808689 B1          & EP 1080780 A1 | 1,3,4<br>2,5 |
| X<br>Y | JP 2001-137688 A  (Nippon Shokubai Co., Ltd.),<br>22 May, 2001 (22.05.01),<br>Par. Nos. [0056], [0057]; examples<br>& US 6808689 B1          & EP 1080780 A1 | 1,3,4<br>2,5 |

| ☒ Further documents are listed in the continuation of Box C. | ☐ See patent family annex. |
| --- | --- |

\* Special categories of cited documents:
"A" document defining the general state of the art which is not considered   to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>    02 October, 2008 (02.10.08) | Date of mailing of the international search report<br>    14 October, 2008 (14.10.08) |
| --- | --- |
| Name and mailing address of the ISA/<br>    Japanese Patent Office | Authorized officer |
| Facsimile No. | Telephone No. |

Form PCT/ISA/210 (second sheet) (April 2007)

**INTERNATIONAL SEARCH REPORT**

International application No.

PCT/JP2008/063472

C (Continuation).   DOCUMENTS CONSIDERED TO BE RELEVANT

| Category* | Citation of document, with indication, where appropriate, of the relevant passages | Relevant to claim No. |
|---|---|---|
| Y<br>A | JP 2006-298797 A   (Nippon Shokubai Co., Ltd.),<br>02 November, 2006 (02.11.06),<br>Examples<br>& US 2006/0235243 A1      & EP 1714955 A1 | 2,5<br>1,3,4 |
| Y<br>A | JP 6-263689 A  (Nippon Shokubai Co., Ltd.),<br>20 September, 1994 (20.09.94),<br>Claims<br>& US 5442108 A           & EP 614872 A1 | 5<br>1-4 |
| X<br>Y | JP 2001-129384 A  (Nippon Shokubai Co., Ltd.),<br>15 May, 2001 (15.05.01),<br>Par. Nos. [0033] to [0035]; examples<br>& US 6994833 B1         & EP 1097745 A1 | 1,3,4<br>2,5 |
| X<br>Y | JP 11-130722 A  (Nippon Shokubai Co., Ltd.),<br>18 May, 1999 (18.05.99),<br>Claims; Par. No. [0032]; examples<br>& US 6069271 A            & EP 911313 A1 | 1,3,4<br>2,5 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP SHO551980102536 A
- JP SHO62198717579 B
- JP HEI11989165543 A
- JP HEI61994262081 A
- JP HEI6263689 A
- JP 2007509884 A
- JP SHO55102536 A
- JP SHO6217579 B

- JP HEI1165543 A
- JP HEI6262081 A
- JP 263689 A
- JP 2003251183 A
- JP HEI81996206505 A
- JP HEI41992217932 A
- JP HEI91997241209 A